(19) Europäisches Patentamt
European Patent Office
Office européen des brevets

(11) Numéro de publication: **0 555 460 B1**

# (12) FASCICULE DE BREVET EUROPEEN

(45) Date de publication de fascicule du brevet: **31.05.95**

(51) Int. Cl.$^{6}$: **A61K 7/42**

(21) Numéro de dépôt: **92919033.8**

(22) Date de dépôt: **25.08.92**

(86) Numéro de dépôt internationale :
**PCT/FR92/00821**

(87) Numéro de publication internationale :
**WO 93/04666 (18.03.93 93/08)**

## (54) COMPOSITION COSMETIOUE FILTRANTE COMPRENANT UN NANOPIGMENT D'OXYDE METALLIOUE ET UN POLYMERE FILTRE LIPOSOLUBLE.

(30) Priorité: **29.08.91 FR 9110731**

(43) Date de publication de la demande:
**18.08.93 Bulletin 93/33**

(45) Mention de la délivrance du brevet:
**31.05.95 Bulletin 95/22**

(84) Etats contractants désignés:
**AT BE CH DE DK ES FR GB GR IE IT LI NL SE**

(56) Documents cités:
**WO-A-90/11067**
**FR-A- 2 622 440**
**FR-A- 2 657 351**

(73) Titulaire: **L'OREAL**
**14, Rue Royale**
**F-75008 Paris (FR)**

(72) Inventeur: **FORESTIER, Serge**
**16, allée Ferdinand-Buisson**
**F-77410 Claye-Souilly (FR)**
Inventeur: **HANSENNE, Isabelle**
**156-158, rue Legendre**
**F-75017 Paris (FR)**

(74) Mandataire: **Casalonga, Axel**
**BUREAU D.A. CASALONGA - JOSSE**
**Morassistrasse 8**
**D-80469 München (DE)**

EP 0 555 460 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet une composition filtrant les radiations ultraviolettes, comprenant en association au moins un nanopigment d'oxyde métallique et au moins un polymère liposoluble filtrant les UV.

On sait que les radiations lumineuses de longueurs d'onde comprises entre 280 et 400 nm permettent le brunissement de l'épiderme humain et que les rayons de longueurs d'onde comprises entre 280 et 320 nm, connus sous la dénomination UV-B, provoquent également des érythèmes et des brûlures cutanées qui peuvent nuire au développement du bronzage.

Toutefois, si les rayons UV-B de longueurs d'onde comprises entre 280 et 320 nm jouent un rôle prépondérant dans la production de l'érythème solaire et doivent être filtrés, il n'en reste pas moins vrai que les rayons UV-A de longueurs d'onde comprises entre 320 et 400 nm provoquant le brunissement de la peau, sont susceptibles d'induire également une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques.

On connaît des composés qui ont la propriété d'absorber l'ultraviolet dans la zone érythémateuse tout en laissant passer les radiations responsables du bronzage et on a déjà proposé l'utilisation de tels composés comme "filtres" solaires dans des compositions cosmétiques favorisant l'acquisition du bronzage de la peau en évitant les brûlures et les irritations de la peau.

On connaît également des polymères carbonés synthétiques ou naturels et des polymères siloxaniques porteurs d'un groupement absorbant l'ultraviolet qui présentent l'avantage de réduire, voire de supprimer la pénétration du composé filtre dans l'organisme.

Les oxydes métalliques tels que l'oxyde de titane sont intéressants du fait de leurs propriétés de diffusion et de réflexion des rayons ultraviolets sur une large bande. Cependant, l'efficacité des compositions cosmétiques renfermant seulement des oxydes métalliques, exprimée par le facteur de protection solaire que l'on convient d'appeler "indice de protection" ou IP, est insuffisante pour des peaux très sensibles ou continuellement exposées au rayonnement solaire, notamment en ce qui concerne l'indice de protection UV-B.

L'indice de protection ou IP peut s'exprimer par le rapport du temps d'irradiation nécessaire pour atteindre le seuil érythématogène avec le filtre UV au temps nécessaire pour atteindre le seuil érythématogène sans filtre UV.

Les compositions cosmétiques ne renfermant que des "polymères filtres" présentent le même inconvénient.

On conviendra d'appeler, dans la suite du texte, "polymère filtre" un polymère de structure hydrocarbonée ou siloxanique porteur d'au moins un groupement absorbant les ultraviolets.

Par ailleurs, sur le plan industriel, il est bien sûr avantageux de disposer de filtres UV qui permettent, à des concentrations faibles, d'obtenir des compositions solaires à indice de protection élevé.

La demanderesse vient de découvrir qu'en associant au moins un nanopigment d'oxyde métallique de granulométrie inférieure à 100 nm avec au moins un polymère filtre liposoluble dans un support cosmétiquement acceptable, on obtenait de façon surprenante, pour une composition renfermant une concentration donnée du nanopigment et du polymère filtre pris en combinaison, un indice de protection, notamment UV-B, largement supérieur aux indices de protection de compositions renfermant, soit un nanopigment, soit un polymère filtre, à la même concentration et dans le même support.

La présente invention a donc pour objet une composition cosmétique filtrant les radiations ultraviolettes, de longueurs d'ondes comprises entre 280 et 400 nm renfermant au moins un nanopigment d'oxyde métallique et au moins un polymère filtre choisi parmi les polymères liposolubles à structure hydrocarbonée et les polymères à structure siloxanique, dans un support cosmétiquement acceptable.

La présente invention a également pour objet un procédé de protection de l'épiderme humain et des cheveux contre les radiations ultraviolettes de longueurs d'onde comprises entre 280 et 400 nm, consistant à appliquer sur la peau une quantité efficace de la composition cosmétique filtrante précitée comprenant en association au moins un nanopigment d'oxyde métallique et au moins un polymère filtre liposoluble.

Les pigments d'oxydes métalliques sont choisis parmi les oxydes de titane, de zinc, de cérium, de zirconium, de fer ou leurs mélanges. Dans la présente demande, on entendra par "nanopigments" des pigments de diamètre moyen inférieur à 100 nanomètres, et de préférence compris entre 5 et 50 nanomètres. Ces nanopigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène des silicones, des protéines (collagène, élastine), des alcanolamines, des oxydes de silicium, des oxydes métalliques ou de l'hexamétaphosphate de sodium.

Les pigments enrobés sont plus particulièrement des oxydes de titane enrobés :

- de silice tel que le produit "SUNVEIL" de la société IKEDA,
- de silice et d'oxyde de fer tel que le produit "SUNVEIL F" de la société IKEDA,
- de silice et d'alumine tels que les produits "MICROTITANIUM DIOXIDE MT 500 SA" et"MICROTITANIUM DIOXIDE MT 100 SA" de la société TAYCA, "TIOVEIL" de la société TIOXIDE,
- d'alumine tels que les produits "TIPAQUE TTO-55 (B)" et "TIPAQUE TTO-55 (A)" de la société ISHIHARA, et" UVT 14/4" de la société KEMIRA,
- d'alumine et de stéarate d'aluminium tel que le produit "MICROTITANIUM DIOXIDE MT 100 T" de la société TAYCA,
- d'alumine et de laurate d'aluminium tel que le produit "MICROTITANIUM DIOXIDE MT 100 S" de la société TAYCA,
- d'oxyde de fer et de stéarate de fer tel que le produit "MICROTITANIUM DIOXIDE MT 100 F" de la société TAYCA,
- d'oxyde de zinc et de stéarate de zinc tel que le produit "BR 351" de de la société TAYCA,
- de silice, d'alumine et de silicone tels que les produits "MICROTITANIUM DIOXIDE MT 600 SAS" et "MICROTITANIUM DIOXIDE MT 500 SAS" de la société TAYCA,
- de silice, d'alumine, de stéarate d'aluminium et de silicone tel que le produit "STT-30-D-S" de la société TITAN KOGYO,
- d'alumine et de silicone tel que le produit "TIPAQUE TTO-55 (S)" de la société ISHIHARA,
- de triéthanolamine tel que le produit "STT-65-S" de la société TITAN KOGYO,
- d'acide stéarique tel que le produit "TIPAQUE TTO-55 (C)" de la société ISHIHARA,
- d'hexamétaphosphate de sodium tel que le produit "MICROTITANIUM DIOXIDE MT 150 W" de la société TAYCA.

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la Société KEMIRA ou enrobé d'alumine, de silice et de glycérine tel que le produit "M 211" vendu par la Société KEMIRA.

Les oxydes de titane non enrobés sont par exemple vendus par la société TAYCA sous les dénominations commerciales "MICROTITANIUM DIOXIDE MT 500 B" ou "MICROTITANIUM DIOXIDE MT 600 B", par la société DEGUSSA sous la dénomination "P 25", par la société WACKHERR sous la dénomination "Oxyde de titane transparent PW", par la société MIYOSHI KASEI sous la dénomination "UFTR" et par la Société TOMEN sous la dénomination "ITS".

Les oxydes de zinc non enrobés sont par exemple vendus par la société SUMITOMO sous la dénomination "ULTRA FINE ZINC OXIDE POWDER", par la société PRESPERSE sous la dénomination "FINEX 25" ou par la société IKEDA sous la dénomination "MZO-25".

L'oxyde de cérium non enrobé est vendu sous la dénomination "COLLOIDAL CERIUM OXIDE" par la société RHONE POULENC.

Les oxydes de fer sont, par exemple, vendus par la Société HILTON DAVIS sous les dénominations "PUR OXY YELLOW HIGH TRANSPARENCY", "PUR OXY RED HIGH TRANSPARENCY" et "PUR OXY BLACK HIGH TRANSPARENCY".

Selon l'invention, les nanopigments d'oxyde de titane, enrobés ou non enrobés, sont particulièrement préférés.

Les polymères filtres à chaîne hydrocarbonée liposolubles utilisés selon l'invention peuvent être :

a) des polymères à structure polyéthylénique éventuellement substituée, de polyéthylèneimine, de chitine ou de chitosane, sur lesquels sont greffées des molécules absorbant le rayonnement ultraviolet par l'intermédiaire d'une fonction ester, amide, éther, thioéther, sulfonyle ou acyle,

b) des polymères résultant de l'homo- ou de la copolymérisation de molécules absorbant le rayonnement ultraviolet ("monomères filtres") portant un groupement insaturé choisi parmi les radicaux : allyle, vinyle, acrylamide méthacrylamide, vinyloxycarbonylméthyle, acrylamidoalkyle et notamment acrylamidométhyle, méthacrylamidoalkyle, acrylamido(phényl)alkyle, méthacrylamido(phényl) alkyle, acryloxy, acryloxyalkyle et acryloxypolyoxyéthylène, avec éventuellement d'autres monomères insaturés.

A titre de molécules absorbant le rayonnement ultraviolet, on peut citer les composés suivants :

- le benzylidène camphre et ses dérivés substitués sur le noyau benzénique
- l'isophtalylidène camphre et le téréphtalylidène camphre éventuellement substitués sur le noyau benzénique
- l'acide cinnamique éventuellement substitué par un ou plusieurs groupes alcoxy inférieurs et ses esters,
- l'acide salicylique et ses esters,
- l'acide benzoïque et ses esters,
- l'acide p-aminobenzoïque et ses dérivés alkylés sur le groupe ment amino et leurs esters,
- les hydroxyhenzophénones éventuellement substituées
- le dibenzoylméthane éventuellement substitué
- le benzotriazole et les 2-arylbenzotriazoles
- les 2 arylbenzimidazoles
- les 2-arylbenzofurannes
- les 2-arylbenzoxazoles
- les 2-arylindoles
- les mono- ou diphénylcyanoacrylates
- les absorbeurs de structure coumarinique.

A titre de monomères insaturés copolymérisables avec les "monomères filtres", on peut citer :

les acides acrylique, méthacrylique, itaconique, crotonique ou leurs esters, l'acrylamide et ses dérivés, le méthacrylamide et ses dérivés, l'acrylonitrile, le méthacrylonitrile, le styrène, l'α-méthylstyrène, l'isoprène, le butadiène, l'éthylène, le propylène, les esters vinyliques, les éthers vinyliques, les chlorures et fluorures de vinyle, le chlorure de vinylidène, la N-vinylpyrrolidone, la N-méthacryloyl D-glucosamine et les monoesters et diesters des acides maléique et fumarique.

Dans la présente invention, on entendra par "polymère liposoluble" un polymère insoluble dans l'eau à une concentration supérieure à 0,1% en poids à température ambiante et soluble ou dispersible dans une huile cosmétique telle que l'adipate d'isopropyle à une concentration d'au moins 1% en poids à température ambiante.

A titre de polymères filtres à chaîne hydrocarbonée liposolubles pouvant être utilisée selon l'invention, on peut citer à titre d'exemples :

1) les polymères comportant des motifs de formule (I) :

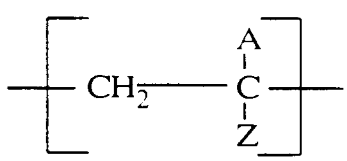 (1)

dans laquelle

Z désigne :

a) - $CONHCH_2X$, A désignant un atonie d'hydrogène et X désignant un groupement aromatique absorbeur d'UV, tels que ceux décrits dans les brevets français n° 2 597 336, 2 237 912, 2 359 857 et 2 596 400, dans lesquels X est de préférence choisi parmi les radicaux benzylidène camphre, éventuellement substitués en positions 3 et 4 par un radical alcoxy en $C_1$-$C_{12}$ ou par un radical méthylènedioxy, les radicaux 2-(2'-hydroxyphényl)benzotriazole éventuellement substitués en position 5' par un radical méthyle ou tert.-octyle, le radical 4-méthoxy 4'-tert.-butyl-dibenzoylméthane, les radicaux 4-hydroxybenzophénone, 2-hydroxybenzophénone éventuellement substituée par un groupement méthoxy en position 4, les radicaux dérivés de 4-hydroxycoumarine et 7-hydroxycoumarine;

b)

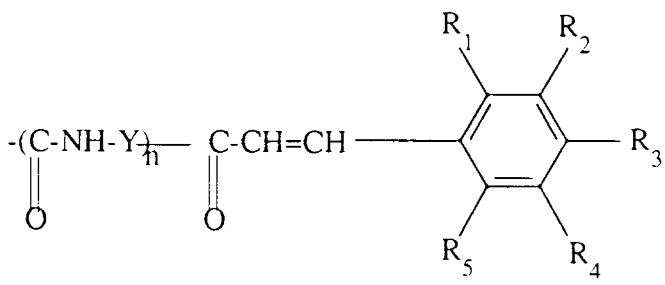

où n est 0 ou 1

lorsque n = 0, A représente un atome d'hydrogène;

lorsque n = 1, A représente un atome d'hydrogène ou un radical méthyle,

$R_1$, $R_2$, $R_3$, $R_4$, $R_5$, identiques ou différents,

représentent un atome d'hydrogène, un radical alkyle linéaire ou ramifié ayant de 1 à 8 atomes de carbone, un radical alcoxy ayant de 1 à 4 atomes de carbone, un radical dialkylamino ou dialkylaminoalkyle de formule :

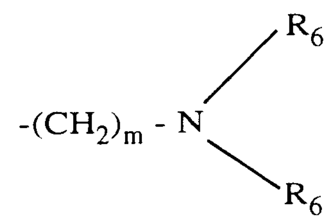

dans laquelle m est 1 à 3 et $R_6$ représente un radical méthyle ou éthyle, lesdits radicaux étant éventuellement quaternisés à l'aide d'un agent de quaternisation pris dans le groupe constitué par le chlorure de méthyle, le bromure de dodécyle, le sulfate de diméthyle et l'acide chloroacétique;

Y étant nul ou pouvant désigner $-CH_2-$, $-CH(CH_3)-$, $>CH-CH_2-CH(CH_3)_2$, $>CH-CH_2-C_6H_5$, $-C(CH_3)_2-CH_2-$, ces polymères étant décrits dans le brevet français n° 2 617 399;

2) les polymères dérivés d'acétate de vinyle comportant le motif de formule (II)

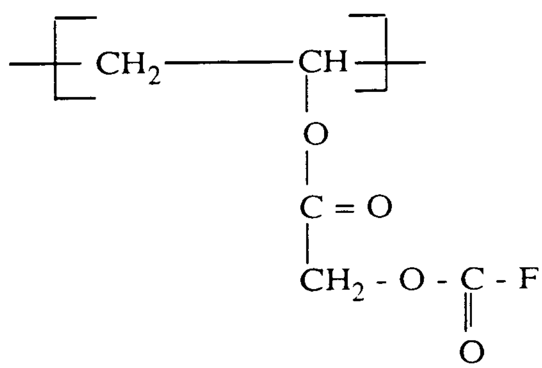

(II)

dans laquelle F représente un radical absorbeur d'UV, tels que ceux décrits dans les brevets français n° 2 197 023 et 2 359 856;

le groupement

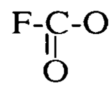

étant choisi de préférence parmi les groupes p-dialkyl($C_1$-$C_4$)aminobenzoate, cinnamate éventuellement substitué par un méthoxy, salicylate, diphénylcyanoacrylate et flufénate ou 3'-trifluorométhyldiphénylamine 2-carboxylate;

3) les copolymères de polyéthylèneimine de poids moléculaire compris entre 500 et 100 000 et de chlorure d'acide para dialkyl ($C_1$-$C_4$) aminobenzoïque tels que ceux décrits dans le brevet US 3 864 473;

4) les copolymères de monomères à insaturations éthyléniques et de dérivés d'acide 4-(N,N-diallylamino)benzoïque ou ses esters tels que ceux décrits dans le brevet US 3 795 733;

5) les produits de polymérisation des monomères de formule

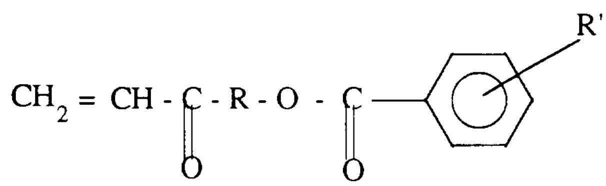

dans laquelle
R désigne

$$-(X)_n-O \quad \text{ou} \quad -(CH_2CH_2-O)_n- \ ,$$

X désignant un radical divalent alkylène, arylène, alkylarylène,
n = 1 à 1000,
R' désigne OH, $N(R_1)_2$,
$R_1$ désigne H, alkyle, aryle, alkylaryle,
les radicaux alkyle ayant de 1 à 20 atomes de carbone,
tels que ceux décrits par exemple dans le brevet WO 88/09783;

6) les polymères dérivés de chitine et de chitosane portant au moins un groupement absorbeur d'UV de formule :

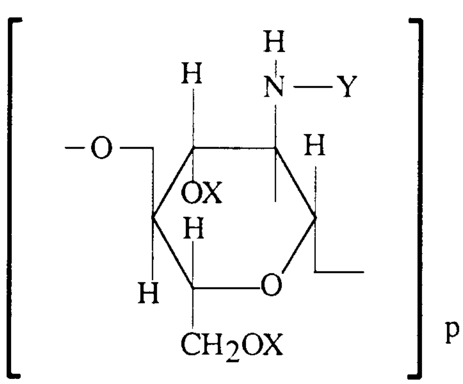 (III)

dans laquelle :
X et Y, indépendamment l'un de l'autre, désignent hydrogène, un radical benzoyle de formule (IV) ou un radical cinnamoyle de formule (V) et Y peut aussi désigner acétyle :

(IV) 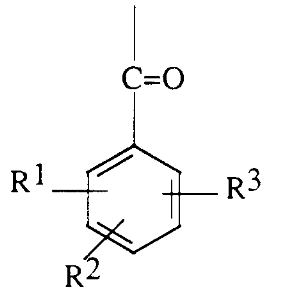 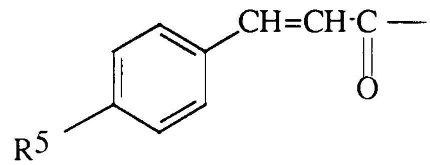 (V)

formules dans lesquelles :

$R^1$, $R^2$ et $R^3$, identiques ou différents, désignent hydrogène, alcoxy en $C_1$-$C_4$, hydroxy ou

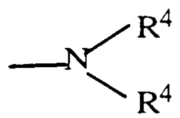

$R^4$ désignant hydrogène, alkyle en $C_1$-$C_4$, monohydroxyalkyle en $C_2$-$C_4$ ou dihydroxyalkyle en $C_2$-$C_4$ sous réserve qu'au moins un des groupements $R^1$, $R^2$ ou $R^3$ ne désigne pas hydrogène et qu'au plus un des groupements $R^1$, $R^2$ ou $R^3$ désigne

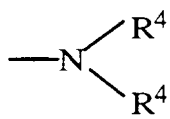

$R^5$ désigne alcoxy en $C_1$-$C_4$

p = 3 - 20 000,

sous réserve que lorsque X désigne hydrogène. Y ne désigne ni hydrogène, ni acétyle;

de tels polymères sont décrits dans la demande de brevet allemand n° 3 912 122.

Les polymères filtres liposolubles à chaîne hydrocarbonée préférés selon l'invention sont les polyacrylamides à greffons X benzylidène camphre éventuellement substitués tels que définis ci-dessus en 1) a).

Les polymères filtres à chaîne siloxanique utilisés selon l'invention sont des diorganopolysiloxanes comportant dans leur molécule au moins une unité de formule :

$$X - \underset{}{\overset{R'_a}{Si}} - O_{\frac{3-a}{2}} \quad (VI)$$

dans laquelle

R' désigne un groupe hydrocarboné saturé ou insaturé en $C_1$-$C_{30}$, un groupe hydrocarboné halogéné en $C_1$-$C_8$ ou un groupe triméthylsilyloxy;

a = 1 ou 2;

X = - A - Y

où A représente un radical divalent hydrocarboné aliphatique ou aromatique comportant au moins 2 atomes de carbone et renfermant éventuellement un ou plusieurs atomes d'oxygène;

Y représente le reste d'une molécule filtrant le rayonnement ultràviolet.

En plus des unités de formule (VI) le diorganopolysiloxane peut comporter des unités de formules :

$$R'_b - Si\,O_{\frac{4-b}{2}} \quad (VII) \quad \text{et} \quad Z - \overset{\overset{\displaystyle R'_a}{|}}{Si} - O_{\frac{3-a}{2}} \quad (VIII)$$

dans lesquelles R' et a ont la même signification que dans la formule (VI);

b est un nombre entier désignant 1, 2 ou 3;

Z = - O - Y , Y ayant la même signification que dans la formule (VI).

A titre de groupe hydrocarboné, ou peut citer les radicaux alkyle en $C_1$-$C_{30}$, alcényle en $C_2$-$C_{30}$, cycloalkyle ou aromatique comme phényle ou tolyle.

A titre de groupe hydrocarboné halogéné, on peut citer le radical 3,3,3,-trifluoropropyle.

Dans le diorganopolysiloxane constitué de motifs (VI) et éventuellement (VII) et (VIII), au moins 40% en nombre des radicaux R' sont des radicaux méthyle. Le nombre total des unités (VI), (VII) et (VIII) est de préférence inférieur ou égal à 250 et est compris en particulier entre 2 et 50.

Y représente de préférence un reste :

- benzylidène camphre éventuellement substitué sur le noyau benzénique par des radicaux hydroxyle, alkyle ou alcoxy en $C_1$-$C_6$;
- benzalmalonate de dialkyle en $C_1$-$C_8$, éventuellement substitué sur le noyau benzénique par des radicaux hydroxy, alkyle ou alcoxy en $C_1$-$C_6$;
- 2-(2'-hydroxyphényl)benzotriazole portant éventuellement sur l'un des noyaux aromatiques des substituants alkyle en $C_1$-$C_8$ ou alcényle en $C_2$-$C_8$, halogène. alcoxy, carboxy, hydroxy, amino ou tétraalkylpipéridyle;
- dibenzoylméthane portant éventuellement des substituants alkyle ou alcoxy en $C_1$-$C_8$ ou hydroxy;
- benzophénone portant éventuellement des substituants alkyle ou alcoxy en $C_1$-$C_8$ ou hydroxy;
- benzoate substitué par des radicaux hydroxy, alcoxy en $C_1$-$C_6$,amino ou mono ou di($C_1$-$C_6$ alkyl)-amino.

De tels polymères filtres à chaîne siloxanique sont décrits dans les demandes de brevet européen n° 0 335 777, 0 392 882, 0 388 218, 0 392 883, 0 383 655 et 0 389 337 et dans les brevets français 2 550 787 et 2 657 351 et dans les brevets américains n° 4 696 969, 4 554 369, 4 562 278, 3 513 184 et 4 859 759.

Les silicones filtres préférées pour être utilisées selon l'invention sont celles dans lesquelles le radical Y désigne un reste benzylidène camphre ou 2-(2'hydroxyphényl)benzotriazole éventuellement substitué tel que défini ci-dessus.

La concentration en nanopigments dans les compositions selon l'invention est comprise entre 0,1 et 15% en poids et de préférence entre 0,5 et 10% en poids par rapport au poids total de la composition.

Le ou les polymères filtres sont présents à une concentration totale comprise entre 0,1 et 15% en poids et de préférence entre 0,5 et 10% en poids par rapport au poids total de la composition.

Le rapport en poids nanopigment(s)/polymère(s) filtre(s) est avantageusement compris entre 0,1 et 10 et de préférence entre 0,5 et 5.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, d'huile, de dispersion vésiculaire, de crème, de lait, de poudre, de bâtonnet solide et éventuellement être conditionnée en aérosol et se présenter sous forme de mousse ou de spray.

Elle peut contenir les adjuvants cosmétiques habituellement utilisés tels que des corps gras, des solvants organiques, des silicones, des épaississants, des adoucissants, des filtres solaires UV-A, UV-B, ou à bande large, des agents anti-mousses, des agents hydratants, des parfums, des conservateurs, des tensio-actifs, des charges, des séquestrants, des polymères anioniques, cationiques, non-ioniques, amphotères ou leurs mélanges, des propulseurs, des agents alcalinisants ou acidifiants, des colorants, des pigments d'oxydes métalliques de granulométrie comprise entre 100 nm et 20 000 nm comme les oxydes de fer, ou tout autre ingrédient habituellement utilisé en cosmétique.

Les corps gras peuvent être constitués par une huile ou une cire ou leur mélange, les acides gras, les alcools gras, la vaseline, la paraffine, la lanoline, la lanoline hydrogénée, la lanoline acétylée.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin, les huiles de silicone et les isoparaffines.

Les cires sont choisies parmi les cires animales, fossiles, végétales minérales ou de synthèse. On peut citer notamment les cires d'abeille, les cires de Carnauba, de Candelila, de canne à sucre, du Japon, les ozokérites, la cire de Montan, les cires microcristallines, les paraffines les cires et résines de silicone.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, ou encore sous forme d'émulsion telle qu'une crème ou un lait, sous forme de pommade, de gel, de bâtonnet solide ou de mousse aérosol.

Les émulsions peuvent contenir en outre des agents tensio-actifs anioniques, non-ioniques, cationiques ou amphotères.

Elle peut se présenter aussi sous forme de dispersion vésiculaire de lipides amphiphiles ioniques ou non-ioniques, préparée selon des procédés connus. On peut, par exemple, faire gonfler les lipides dans une solution aqueuse pour former des sphérules dispersées dans le milieu aqueux comme décrit dans l'article BANGHAM, STANDISH & WATKINS, J. mol. Biol., 13, 238 (1965) ou dans les brevets FR-2 315 991 et 2 416 008 de la demanderesse.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffants ou traitants, de lotion ou gel pour le brushing ou la mise en plis, de laque pour cheveux, de composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encre appelé "eye liner", elle peut se présenter sous forme solide ou pâteuse, anhydre ou aqueuse, comme des émulsions huile-dans-eau ou eau-dans-huile, des dispersions vésiculaires ou encore des suspensions.

L'invention a également pour objet un procédé de protection de l'épiderme humain et des cheveux contre le rayonnement ultraviolet consistant à appliquer sur la peau ou les cheveux une quantité efficace de la composition cosmétique ci-dessus.

L'invention sera mieux illustrée par les exemples non limitatifs ci-après.

EXEMPLE 1

On prépare une crème solaire de composition suivante :

| | | |
|---|---|---|
| - Oxyde de titane enrobé d'oxyde de fer et de stéarate de fer (diamètre moyen 15 nm) vendu sous la dénomination "MICRO TITANIUM DIOXIDE MT 100F" par la société TAYCA | | 5 g |
| - Polydiméthylsiloxane à greffon 2-(3'-triméthylène-5'-méthyl-2'-hydroxyphényl)-benzotriazole de formule : | | 5 g |

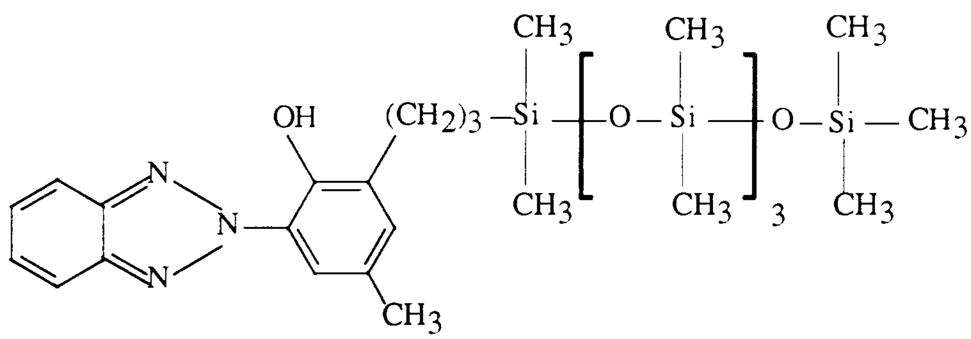

| | | |
|---|---|---|
| selon l'exemple 1 de la demande EP 0388 218 | | |
| - Mélange de stéarate de glycérol et de stéarate de polyéthylène glycol à 100 moles d'oxyde d'éthylène vendu sous la dénomination "ARLACEL 165" par la société ICI | | 1 g |
| - Acide isostéarique | | 2 g |
| - 2-octyldodécanol | | 15 g |
| - Alcool stéarylique | | 1 g |
| - Glycérine | | 3 g |
| - Sorbitol en solution aqueuse à 70% de MA | | 1,4 g MA |
| - Acide polyacrylique réticulé vendu sous la dénomination "CARBOPOL 940" par la société GOODRICH | | 0,3 g |
| - Triéthanolamine | | 0,4 g |
| - Conservateurs, antioxydant | qs | |
| - Parfum | qs | |
| - Eau | qsp | 100 g |

EXEMPLE 2

On prépare une crème solaire de composition suivante :

| | | |
|---|---|---|
| - Oxyde de titane enrobé d'oxyde de fer et de stéarate de de fer (diamètre moyen 15 nm) vendu sous la dénomination "MICROTITANIUM DIOXIDE MT 100F" par la société TAYCA | | 5 g |
| - Poly(4'-acrylamidométhyl-3-benzylidènecamphre) de l'exemple 1 du brevet français n° 2 597 336 constitué d'unités : 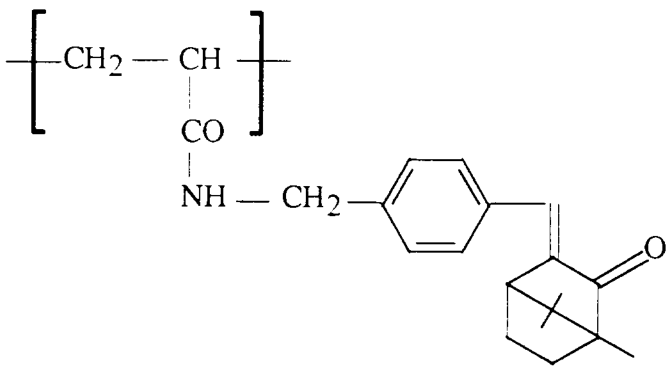 | | 5 g |
| - Mélange de stéarate de glycérol et de stéarate de polyéthylène glycol à 100 moles d'oxyde d'éthylène vendu sous la dénomination "ARLACEL 165" par la société ICI | | 1 g |
| - Acide isostéarique | | 2 g |
| - 2-octyldodécanol | | 15 g |
| - Alcool stéarylique | | 1 g |
| - Glycérine | | 3 g |
| - Sorbitol en solution aqueuse à 70% de MA | | 1,4 g MA |
| - Acide polyacrylique réticulé vendu sous la dénomination "CARBOPOL 940" par la société GOODRICH | | 0,3 g |
| - Triéthanolamine | | 0,4 g |
| - Conservateurs, antioxydant | qs | |
| - Parfum | qs | |
| - Eau | qsp | 100 g |

EXEMPLE 3

On prépare une émulsion solaire H/E de composition suivante :

| | | | |
|---|---|---|---|
| - Mélange de stéarate de glycérol et de stéarate de polyéthylèneglycol à 100 moles d'oxyde d'éthylène vendu sous la dénomination "ARLACEL 165" par la Société ICI | 2 | g | |
| - Huile de vaseline | 8 | g | |
| - Acide stéarique | 2 | g | |
| - Lanoline | 2 | g | |
| - Alcool stéarylique | 1 | g | |
| - Polydiméthylsiloxane à greffons 2-(3'-triméthylène-2'-hydroxy-5'-méthylphényl)benzotriazole selon la demande EP 0392 883 | 2 | g | |

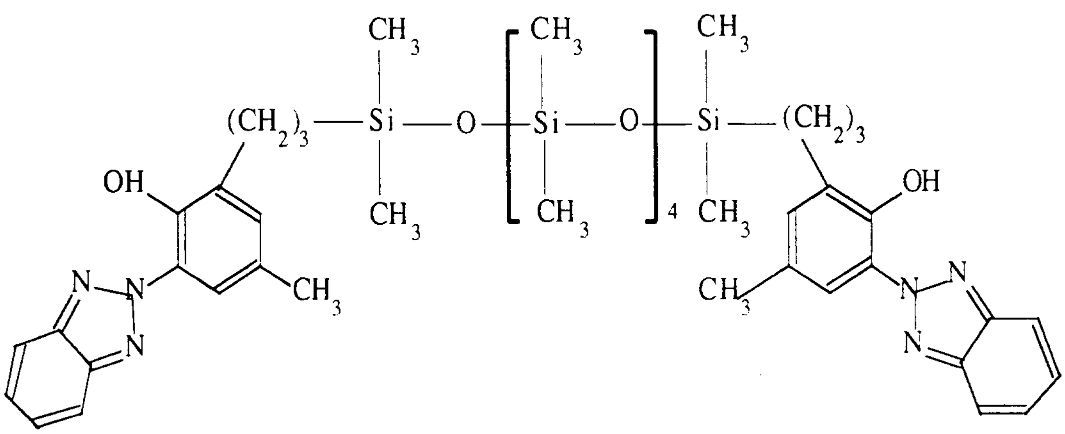

| | | | |
|---|---|---|---|
| - Glycérine | 3 | g | |
| - Sorbitol en solution aqueuse à 70% de MA | 1,4 | g | MA |
| - Hydroxyéthylcellulose modifiée par une chaîne cétyle vendue sous la dénomination "NATROSOL PLUS GRADE 330 CS" par la Société AQUALON | 0,5 | g | |
| - Hexadécylphosphate de potassium | 0,5 | g | |
| - Acétate de sodium monohydraté | 0,2 | g | |
| - Oxyde de cérium colloïdal vendu en solution aqueuse à 20% de MA par la Société RHONE POULENC sous la dénomination "COLLOIDAL CERIUM OXIDE" | 2 | g | MA |

- Conservateurs qs
- Eau qsp 100 g

EXEMPLE 4

On prépare une émulsion solaire H/E de composition suivante :

- Mélange de stéarate de glycérol et de stéarate de polyéthylèneglycol à 100 moles d'oxyde d'éthylène vendu sous la dénomination "ARLACEL 165" par la Société ICI 2 g
- Huile de vaseline 8 g
- Acide stéarique 2 g
- Lanoline 2 g
- Alcool stéarylique 1 g
- Mélange de polydiméthylsiloxanes à greffons 4'-triméthylèneoxy 3-benzylidènecamphre et 4'-oxy 3-benzylidènecamphre, préparé selon la demande EP 0335 777, ayant pour formule :

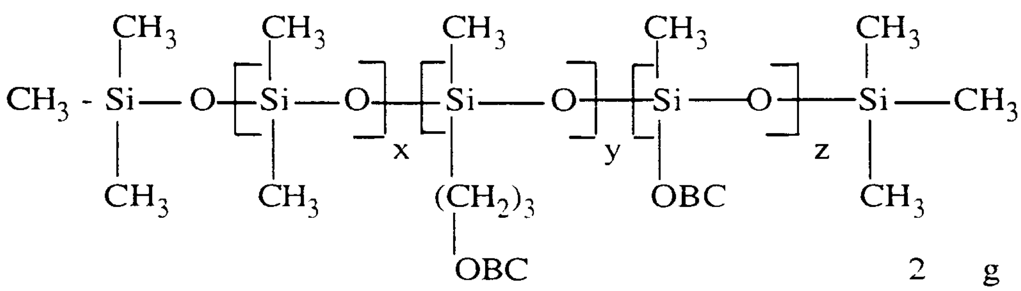

2 g

constitué d'environ :

- 55% de polydiméthylsiloxane dans lequel x = 0 à 20, y = 1 à 4, z = 0
- 5% de polydiméthylsiloxane dans lequel x = 0 à 21, y = 0, z = 1 à 4
- 40% de polydiméthylsiloxane dans lequel x = 0 à 16, y = 1 à 3, z = 1 à 3,

  BC désignant le radical 3-benzylidènecamphre
- Glycérine 3 g
- Sorbitol en solution aqueuse à 70% de MA 1,4 g MA
- Hydroxyéthylcellulose modifiée par une chaîne cétyle vendue sous la dénomination "NATROSOL

| | | | |
|---|---|---|---|
| PLUS GRADE 330 CS" par la Société AQUALON | | 0,5 | g |
| - Hexadécylphosphate de potassium | | 0,5 | g |
| - Acétate de sodium monohydraté | | 0,2 | g |
| - Oxyde de titane enrobé d'oxyde de fer et de stéarate de fer (diamètre moyen 15 nm) vendu sous la dénomination "MICROTITANIUM DIOXIDE MT 100F" par la Société TAYCA | | 2,5 | g |
| - Conservateurs | qs | | |
| - Eau | qsp | 100 | g |

EXEMPLE 5

On prépare une émulsion solaire H/E de composition suivante :

| | | |
|---|---|---|
| - Mélange de stéarate de glycérol et de stéarate de polyéthylèneglycol à 100 moles d'oxyde d'éthylène vendu sous la dénomination "ARLACEL 165" par la Société ICI | 2 | g |
| - Huile de vaseline | 8 | g |
| - Acide stéarique | 2 | g |
| - Lanoline | 2 | g |
| - Alcool stéarylique | 1 | g |
| - Polydiméthylsiloxane à greffon dérivé de benzophénone de formule : | 2 | g |

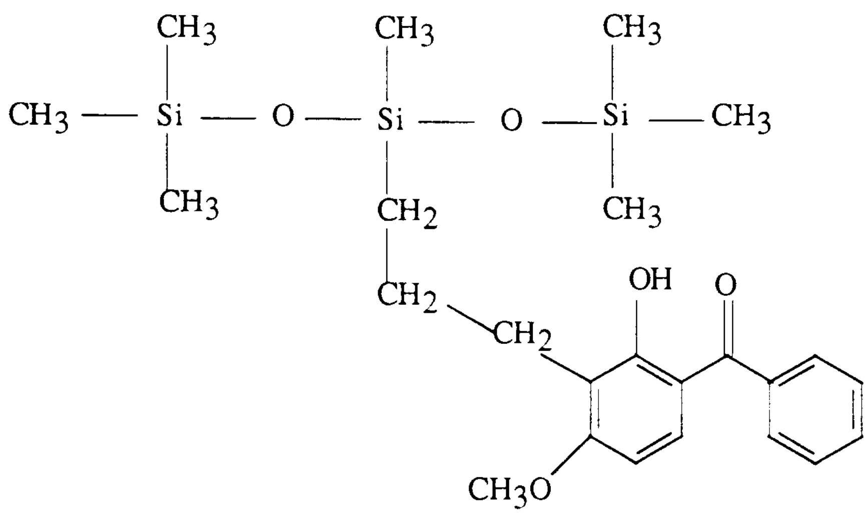

| | | | |
|---|---|---|---|
| - Glycérine | | 3 g | |
| - Sorbitol en solution aqueuse à 70% de MA | | 1,4 g | MA |
| - Hydroxyéthylcellulose modifiée par une chaîne cétyle vendue sous la dénomination "NATROSOL PLUS GRADE 330 CS" par la Société AQUALON | | 0,5 g | |
| - Hexadécylphosphate de potassium | | 0,5 g | |
| - Acétate de sodium monohydraté | | 0,2 g | |
| - Oxyde de zinc vendu sous la dénomination "ULTRA FINE ZINC OXIDE POWDER" par la Société SUMITOMO | | 2 g | |
| - Conservateurs | qs | | |
| - Eau | qsp | 100 g | |

EXEMPLE 6

On prépare un gel crème de composition suivante :

| | |
|---|---|
| - Glycérine | 3 g |
| - Huile de vaseline | 5 g |
| - Emulsion huile-dans-eau de copolymère réticulé d'acrylamide/2-acrylamido 2-méthylpropane sulfonate de sodium, vendue par la Société SEPPIC sous la dénomination "SEPIGEL 305" à 40% de MA en copolymère | 1,2 g MA en copolymère |
| - Oxyde de titane enrobé d'oxyde de fer et de stéarate de fer (diamètre moyen 15 nm) vendu sous la dénomination "MICROTITANIUM DIOXIDE MT 100 F" par la Société TAYCA | 1,33 g |
| - Polydiméthylsiloxane à greffon dérivé de benzophénone de formule : | 2,67 g |

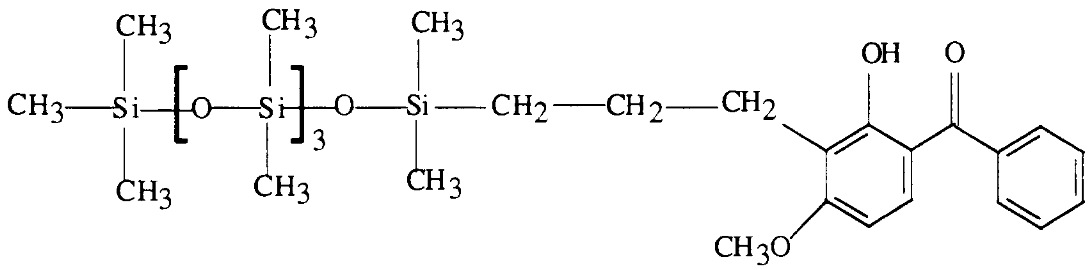

selon la demande de brevet français FR 2 657 351

| | | |
|---|---|---|
| - Mélange de diméthiconol (13%), d'octaméthylcyclotétrasiloxane et de décaméthylcyclopentasiloxane (87%), vendu sous la dénomination "Q2-1401" par la Société DOW CORNING | | 5 g |
| - Polyméthacrylate de méthyle en poudre (15 μ) vendu sous la dénomination "MICROPEARL M 100" par la Société SEPPIC | | 3 g |
| - Conservateurs | qs | |
| - Eau | qsp | 100 g |

## Revendications

1. Composition cosmétique filtrante caractérisée par le fait qu'elle comprend, dans un support cosmétiquement acceptable, au moins un nanopigment d'oxydes métalliques choisis parmi les oxydes de titane, de zinc, de cérium, de zirconium, de fer ou leurs mélanges, de diamètre moyen inférieur à 100 nm, et au moins un polymère porteur d'au moins un groupement absorbant les ultraviolets choisi parmi les polymères à structure hydrocarbonée liposolubles et les polymères à structure siloxanique.

2. Composition cosmétique selon la revendication 1, caractérisée par le fait que les nanopigments d'oxydes métalliques ont un diamètre compris entre 5 et 50 nm.

3. Composition cosmétique selon la revendication 1 ou 2, caractérisée par le fait que l'oxyde métallique est l'oxyde de titane.

4. Composition cosmétique selon l'une quelconque des revendication 1 à 3, caractérisée par le fait que le nanopigment d'oxydes métalliques est un pigment enrobé ayant subi un ou plusieurs traitements de surface de nature chimique, électronique, mécanochimique ou mécanique avec des composés choisis parmi les aminoacides, la cire d'abeille, les acides gras, les alcools gras, les tensio-actifs anioniques, les lécithines, les sels de sodium, potassium, zinc, fer ou aluminium d'acides gras, les alcoxydes métalliques, le polyéthylène, les silicones, les protéines, les alcanolamines, les oxydes de silicium, les oxydes métalliques et l'hexamétaphosphate de sodium.

5. Composition cosmétique selon la revendication 4, caractérisée par le fait que le nanopigment d'oxydes métalliques enrobé est un pigment d'oxyde de titane enrobé de silice, de silice et d'alumine, de silice et d'oxyde de fer, d'alumine et de silicone, d'alumine, d'alumine et de stéarate d'aluminium, d'alumine et de laurate d'aluminium, d'oxyde de fer et de stéarate de fer, d'oxyde de zinc et de stéarate de zinc, de silice et d'alumine et de silicone, de silice et d'alumine et de stéarate d'aluminium et de silicone, de triéthanolamine, d'acide stéarique ou d'hexamétaphosphate de sodium.

**6.** Composition cosmétique selon l'une quelconque des revendications 1 à 5, caractérisée par le fait qu'elle contient 0,1 à 15% en poids, et de préférence 0,5 à 10% en poids par rapport au poids total de la composition, d'au moins un nanopigment d'oxydes métalliques.

**7.** Composition cosmétique selon l'une quelconque des revendications 1 à 6, caractérisée par le fait que les polymères de structure hydrocarbonée liposolubles sont des polymères à structure polyéthylénique éventuellement substituée, de polyéthylèneimine, de chitine ou de chitosane, sur lesquels sont greffées des molécules absorbant le rayonnement ultraviolet par l'intermédiaire d'une fonction ester, amide, éther, thioéther, sulfonyle ou acyle.

**8.** Composition cosmétique selon l'une quelconque des revendications 1 à 6, caractérisée par le fait que les polymères de structure hydrocarbonée liposolubles sont des polymères résultant de l'homo- ou de la copolymérisation de molécules absorbant le rayonnement ultraviolet portant un groupement insaturé choisi parmi les radicaux : allyle, vinyle, acrylamide, méthacrylamide, vinyloxycarbonylméthyle,acrylamidoalkyle et notamment acrylamidométhyle, méthacrylamidoalkyle, acrylamido(phényl)alkyle, méthacrylamido(phényl)alkyle, acryloxy, acryloxyalkyle et acryloxypolyoxyéthylène.

**9.** Composition cosmétique selon la revendication 8, caractérisée par le fait que les polymères à structure hydrocarbonée liposolubles résultent de l'homo- ou de la copolymérisation de molécules absorbant le rayonnement UV portant un groupe insaturé avec d'autres monomères insaturés choisis parmi les acides acrylique, méthacrylique, itaconique, crotonique ou leurs esters, l'acrylamide et ses dérivés, le méthacrylamide et ses dérivés, l'acrylonitrile, le méthacrylonitrile, le styrène, l'α-méthylstyrène, l'isoprène, le butadiène, l'éthylène, le propylène, les esters vinyliques, les chlorures et fluorures de vinyle, le chlorure de vinylidène, la N-vinylpyrrolidone, la N-méthacryloyl D-glucosamine et les monoesters et diesters des acides maléique et fumarique.

**10.** Composition cosmétique selon l'une quelconque des revendications 7 à 9, caractérisée par le fait que les molécules absorbant le rayonnement ultraviolet sont choisies parmi : le benzylidène camphre, et ses dérivés substitués sur le noyau benzénique, l'isophtalylidène camphre et le téréphtalylidène camphre éventuellement substitués sur le noyau benzénique, l'acide cinnamique éventuellement substitué par un ou plusieurs groupes alcoxy inférieurs et ses esters, l'acide salicylique et ses esters, l'acide benzoïque et ses esters, l'acide p-aminobenzoïque et ses dérivés alkylés sur le groupement amino et leurs esters, les hydroxybenzophénones éventuellement substituées, le dibenzoylméthane éventuellement substitué, le benzotriazole et les 2-arylbenzotriazoles, les 2 arylbenzimidazoles, les 2-arylbenzofurannes, les 2-arylbenzoxazoles, les 2-arylindoles, les mono- ou diphénylcyanoacrylates et les absorbeurs de structure coumarinique.

**11.** Composition cosmétique selon la revendication 8, caractérisée par le fait qu'elle comprend un poly(4'-acrylamidométhyl-3-benzylidènecamphre).

**12.** Composition cosmétique selon l'une quelconque des revendications 1 a 11, caractérisée par le fait qu'elle contient un polymère à structure siloxanique constitué par un diorganopolysiloxane comportant dans sa molécule au moins une unité de formule :

$$X - \overset{R'_a}{\overset{|}{Si}} - O_{\frac{3-a}{2}} \quad (VI)$$

dans laquelle

R' désigne un groupe hydrocarboné saturé ou insaturé en $C_1$-$C_{30}$, un groupe hydrocarboné halogéné en $C_1$-$C_8$ ou un groupe triméthylsilyloxy;

a = 1 ou 2;

X = - A - Y

où A est un radical divalent hydrocarboné aliphatique ou aromatique comportant au moins 2 atomes de carbone et renfermant éventuellement un ou plusieurs atomes d'oxygène et

Y représente le reste d'une molécule filtrant le rayonnement ultraviolet.

**13.** Composition cosmétique selon la revendication 12, caractérisée par le fait que le diorganopolysiloxane comporte en outre des unités ayant pour formules :

$$R'_b - Si\,O_{\frac{4-b}{2}} \quad (VII) \quad \text{et} \quad Z - \overset{\overset{\displaystyle R'_a}{|}}{Si} - O_{\frac{3-a}{2}} \quad (VIII)$$

dans lesquelles R' et a ont les significations indiquées dans la revendication 12, b est un nombre entier égal à 1,2 ou 3, Z = - O - Y, Y ayant la même signification que dans la revendication 12, au moins 40% en nombre des radicaux R' désignant méthyle.

**14.** Composition cosmétique selon la revendication 12 ou 13, caractérisée par le fait que le reste Y d'une molécule filtrant le rayonnement ultraviolet est un reste de benzylidène camphre non substitué ou substitué sur le noyau benzénique par un radical hydroxyle, alkyle ou alcoxy en $C_1$-$C_6$; benzalmalonate de dialkyle en $C_1$-$C_8$, non substitué ou substitué sur le noyau benzénique par des radicaux hydroxy, alkyle ou alcoxy en $C_1$-$C_6$; 2-(2'-hydroxyphényl)benzotriazole non substitué ou portant sur l'un des noyaux aromatiques des substituants alkyle en $C_1$-$C_8$, alcényle en $C_2$-$C_8$, halogène, alcoxy, carboxy hydroxy ou amino; dibenzoylméthane non substitué ou portant des substituants alkyle ou alcoxy en $C_1$-$C_8$ ou hydroxy; benzophénone non substituée ou portant des substituants alkyle ou alcoxy en $C_1$-$C_8$ ou hydroxy; benzoate substitué par des radicaux hydroxy, alcoxy en $C_1$-$C_6$, amino ou mono- ou di($C_1$-$C_6$-alkyl)amino.

**15.** Composition cosmétique selon l'une quelconque des revendications 12 à 14, caractérisée par le fait qu'elle comprend un polydiméthylsiloxane à greffon(s) 2-(3'-triméthylène-5'-méthyl-2'-hydroxyphényl)-benzotriazole.

**16.** Composition cosmétique selon l'une quelconque des revendications 12 à 14, caractérisée par le fait qu'elle comprend un mélange de polydiméthylsiloxanes à greffons 4'-triméthylèneoxy-3-benzylidène camphre et 4'-oxy-3-benzylidène camphre.

**17.** Composition cosmétique selon l'une quelconque des revendications 1 à 16, caractérisée par le fait qu'elle contient 0,1 à 15% en poids, et de préférence 0,5 à 10% en poids, par rapport au poids total de la composition d'au moins un polymère filtre liposoluble à structure hydrocarbonée ou siloxanique.

**18.** Composition cosmétique selon l'une quelconque des revendications 1 à 17, caractérisée par le fait que le rapport en poids nanopigment(s)/polymère(s) filtre(s) est compris entre 0,1 et 10 et de préférence entre 0,5 et 5.

**19.** Composition cosmétique selon l'une quelconque des revendications 1 à 18, caractérisée par le fait qu'elle constitue une composition protectrice de l'épiderme humain ou antisolaire et se présente sous forme de lotion, lotion épaissie, gel huile, dispersion vésiculaire, crème, lait, poudre, bâtonnet solide, mousse ou spray.

**20.** Composition cosmétique selon l'une quelconque des revendications 1 à 18, caractérisée par le fait qu'elle constitue une composition de maquillage des cils, des sourcils ou de la peau et se présente sous forme solide ou pâteuse, anhydre ou aqueuse, d'émulsion, de suspension, de dispersion vésiculaire.

**21.** Composition cosmétique selon l'une quelconque des revendications 1 à 18, utilisée pour la protection des cheveux contre les rayons ultraviolets, caractérisée par le fait qu'elle se présente sous forme de shampooing, de lotion, de gel ou composition à rincer, a appliquer avant ou après shampooing, avant ou après coloration ou décoloration, avant, pendant ou après permanente ou défrisage, sous forme de lotion ou de gel coiffants ou traitants, de lotion ou gel pour le brushing ou la mise en plis, de laque pour cheveux, de composition de permanente ou de défrisage, de coloration ou décoloration des cheveux.

22. Composition cosmétique selon l'une quelconque des revendications 19 à 21, caractérisée par le fait qu'elle comprend en outre des adjuvants cosmétiques choisis parmi les corps gras, les solvants organiques, les silicones, les épaississants, les adoucissants, les filtres solaires UV-A, UV-B ou à bande large, les agents antimousses, les agents hydratants, les parfums, les conservateurs, les tensio-actifs, les charges, les séquestrants, les polymères anioniques, cationiques, non-ioniques, amphotères ou leurs mélanges, les propulseurs, les agents alcalinisants ou acidifiants, les colorants et les pigments d'oxydes métalliques de granulométrie comprise entre 100 nm et 20 000 nm.

23. Procédé cosmétique de protection de l'épiderme humain et des cheveux contre le rayonnement ultraviolet de longueurs d'onde comprises entre 280 et 400 nm, caractérisé par le fait qu'il consiste à appliquer sur la peau ou les cheveux une quantité efficace d'une composition cosmétique selon l'une quelconque des revendications 1 à 22.

**Claims**

1. Screening cosmetic composition characterized by the fact that it comprises, in a cosmetically acceptable carrier, at least one nanopigment of metallic oxides chosen from titanium, zinc, cerium, zirconium or iron oxides or mixtures thereof, with a mean diameter of less than 100 nm, and at least one polymer carrying at least one ultraviolet-absorbing group chosen from fat-soluble polymers with hydrocarbon structure and polymers with a siloxane structure.

2. Cosmetic composition according to Claim 1, characterized by the fact that the nanopigments of metallic oxides have a diameter of between 5 and 50 nm.

3. Cosmetic composition according to Claim 1 or 2, characterized by the fact that the metallic oxide is titanium oxide.

4. Cosmetic composition according to any one of Claims 1 to 3, characterized by the fact that the nanopigment of metallic oxides is a coated pigment having undergone one or more surface treatments of a chemical, electronic, mechanicochemical or mechanical nature with compounds chosen from amino acids, beeswax, fatty acids, fatty alcohols, anionic surfactants, lecithins, sodium, potassium, zinc, iron or aluminum salts of fatty acids, metallic alkoxides, polyethylene, silicones, proteins, alkanolamines, silicon oxides, metallic oxides and sodium hexametaphosphate.

5. Cosmetic composition according to Claim 4, characterized by the fact that the coated nanopigment of metallic oxides is a pigment of titanium oxide coated with silica, with silica and alumina, with silica and iron oxide, with alumina and silicone, with alumina, with alumina and aluminum stearate, with alumina and aluminum laurate, with iron oxide and iron stearate, with zinc oxide and zinc stearate, with silica and alumina and silicone, with silica and alumina and aluminum stearate and silicone, with triethanolamine, with stearic acid or with sodium hexametaphosphate.

6. Cosmetic composition according to any one of Claims 1 to 5, characterized by the fact that it contains 0.1 to 15% by weight, and preferably 0.5 to 10% by weight relative to the total weight of the composition, of at least one nanopigment of metallic oxides.

7. Cosmetic composition according to any one of Claims 1 to 6, characterized by the fact that the fat-soluble polymers of hydrocarbon structure are polyethyleneimine, chitin or chitosan polymers with an optionally substituted polyethylenic structure on which are grafted ultraviolet radiation-absorbing molecules via an ester, amide, ether, thioether, sulfonyl or acyl functional group.

8. Cosmetic composition according to one of Claims 1 to 6, characterized by the fact that the fat-soluble polymers of hydrocarbon structure are polymers resulting from the homo- or copolymerization of ultraviolet radiation-absorbing molecules carrying an unsaturated group chosen from the radicals: allyl, vinyl, acrylamide, methacrylamide, vinyloxycarbonylmethyl, acrylamidoalkyl and especially acrylamidomethyl, methacrylamidoalkyl, acrylamido(phenyl)alkyl, methacrylamido(phenyl)alkyl, acryloyloxy, acryloyloxyalkyl and acryloyloxypolyoxyethylene.

**9.** Cosmetic composition according to Claim 8, characterized by the fact that the fat-soluble polymers with hydrocarbon structure result from the homo- or copolymerization of UV radiation-absorbing molecules carrying an unsaturated group, with other unsaturated monomers chosen from acrylic, methacrylic, itaconic and crotonic acids or their esters, acrylamide and its derivatives, methacrylamide and its derivatives, acrylonitrile, methacrylonitrile, styrene, $\alpha$-methylstyrene, isoprene, butadiene, ethylene, propylene, vinyl esters, vinyl chlorides and fluorides, vinylidene chloride, N-vinylpyrrolidone, N-methacryloyl-D-glucosamine and monoesters and diesters of maleic and fumaric acids.

**10.** Cosmetic composition according to any one of Claims 7 to 9, characterized by the fact that the ultraviolet radiation-absorbing molecules are chosen from: benzylidenecamphor and its derivatives substituted on the benzene nucleus, isophthalylidenecamphor and terephthalylidenecamphor, optionally substituted on the benzene nucleus, cinnamic acid, optionally substituted by one or more lower alkoxy groups and its esters, salicylic acid and its esters, benzoic acid and its esters, p-aminobenzoic acid and its derivatives which are alkylated on the amino group and their esters, optionally substituted hydroxybenzophenones, optionally substituted dibenzoylmethane, benzotriazole and 2-arylbenzotriazoles, 2-arylbenzimidazoles, 2-arylbenzofurans, 2-arylbenzoxazoles, 2-arylindoles, mono- or diphenylcyanoacrylates and absorbers of coumarin structure.

**11.** Cosmetic composition according to Claim 8, characterized by the fact that it comprises a poly(4'-acylamidomethyl-3-benzylidenecamphor).

**12.** Cosmetic composition according to any one of Claims 1 to 11, characterized by the fact that it contains a polymer with siloxane structure consisting of a diorganopolysiloxane containing in its molecule at least one unit of formula:

$$X-\overset{\overset{\displaystyle R'_a}{|}}{Si}-O_{\frac{3-a}{2}} \qquad \text{(VI)}$$

in which

R' denotes a saturated or unsaturated $C_1$-$C_{30}$ hydrocarbon group, a halogenated $C_1$-$C_8$ hydrocarbon group or a trimethylsilyloxy group;

a = 1 or 2;

X = -A-Y

where A represents a divalent aliphatic or aromatic hydrocarbon radical containing at least 2 carbon atoms and optionally containing one or more oxygen atoms and

Y represents the residue of an ultraviolet radiation-screening molecule.

**13.** Cosmetic composition according to Claim 12, characterized by the fact that the diorganopolysiloxane contains, in addition, units having the formulae:

$$R'_b-SiO_{\frac{4-b}{2}} \quad \text{(VII) and} \quad Z-\overset{\overset{\displaystyle R'_a}{|}}{Si}-O_{\frac{3-a}{2}} \quad \text{(VIII)}$$

in which R' and a have the meanings indicated in Claim 12, b is an integer equal to 1, 2 or 3, Z = -O-Y, Y having the same meaning as in Claim 12, at least 40% in numerical terms of the R' radicals denoting methyl.

**14.** Cosmetic composition according to Claim 12 or 13, characterized by the fact that the Y residue of an ultraviolet radiation-screening molecule is a benzylidenecamphor residue which is unsubstituted or

substituted on the benzene nucleus by a hydroxyl, $C_1$-$C_6$ alkyl or alkoxy radical; a $C_1$-$C_8$ dialkyl benzalmalonate residue which is unsubstituted or substituted on the benzene nucleus by hydroxyl, $C_1$-$C_6$ alkyl or alkoxy radicals; a 2-(2'-hydroxyphenyl)benzotriazole residue which is unsubstituted or carrying on one of the aromatic nuclei $C_1$-$C_8$ alkyl, $C_2$-$C_8$ alkenyl, halogen, alkoxy, carboxy, hydroxy or amino substituents; a dibenzoylmethane residue which is unsubstituted or carrying $C_1$-$C_8$ alkyl or alkoxy or hydroxy substituents; a benzophenone residue which is unsubstituted or carrying $C_1$-$C_8$ alkyl or alkoxy or hydroxy substituents; a benzoate residue which is substituted by hydroxy, $C_1$-$C_8$ alkoxy, amino or mono- or di($C_1$-$C_6$ alkyl)amino radicals.

**15.** Cosmetic composition according to any one of Claims 12 to 14, characterized by the fact that it comprises a polydimethylsiloxane with 2-(3'-trimethylene-5'-methyl-2'-hydroxyphenyl)benzotriazole graft unit(s).

**16.** Cosmetic composition according to any one of Claims 12 to 14, characterized by the fact that it comprises a mixture of polydimethylsiloxanes with 4'-trimethyleneoxy-3-benzylidenecamphor and 4'-oxy-3-benzylidenecamphor graft units.

**17.** Cosmetic composition according to any one of Claims 1 to 16, characterized by the fact that it contains 0.1 to 15% by weight, and preferably 0.5 to 10% by weight, relative to the total weight of the composition, of at least one fat-soluble screening polymer with hydrocarbon or siloxane structure.

**18.** Cosmetic composition according to any one of Claims 1 to 17, characterized by the fact that the nanopigment(s)/screening polymer(s) ratio by weight is between 0.1 and 10 and preferably between 0.5 and 5.

**19.** Cosmetic composition according to any one of Claims 1 to 18, characterized by the fact that it constitutes a composition for protecting the human epidermis or an anti-sun composition and is provided in the form of a lotion, a thickened lotion, a gel, an oil, a vesicular dispersion, a cream, a milk, a powder, a solid stick, a foam or a spray.

**20.** Cosmetic composition according to any one of Claims 1 to 18, characterized by the fact that it constitutes a make-up composition for the eyelashes, eyebrows or skin and is provided in solid or pasty, anhydrous or aqueous form, as emulsion, suspension or vesicular dispersion.

**21.** Cosmetic compositin according to any one of Claims 1 to 18, which is used for protecting the hair against ultraviolet rays, characterized by the fact that it is provided in the form of a rinse-off shampoo, lotion, gel or composition to be applied before or after shampooing, before or after dyeing or bleaching, before, during or after permenent waving or hair straightening, a hair styling or treatment lotion or gel, a lotion or gel for blow drying or hair setting, a hair lacquer, a composition for permanent waving or hair straightening, and for dyeing or bleaching the hair.

**22.** Cosmetic composition according to any one of Claims 19 to 21, characterized by the fact that it comprises, in addition, cosmetic adjuvants chosen from fatty substances, organic solvents, silicones, thickeners, emollients, UV-A, UV-B or broad band sun-screen agents, antifoaming agents, moisturizing agents, perfumes, preservatives, surfactants, fillers, sequestrants, anionic, cationic, nonionic and amphoteric polymers or mixtures thereof, propellants, alkalinizing or acidifying agents, colorants, pigments of metallic oxides with a particle size of between 100 nm and 20,000 nm.

**23.** Cosmetic process for protecting the human epidermis and the hair against ultraviolet radiation of wawelengths of between 280 and 400 nm, characterized by the fact that it consists in applying to the skin or the hair an effective quantity of a cosmetic composition according to any one of Claims 1 to 22.

**Patentansprüche**

**1.** Kosmetische Filter-Zusammensetzung,
dadurch **gekennzeichnet**, daß
sie, in einem kosmetisch geeigneten Trägermedium, mindestens ein Nanopigment von Metalloxiden, ausgewählt aus Oxiden des Titans, Zinks, Cers, Zirkons, Eisens oder aus deren Mischungen, mit einem

mittleren Durchmesser von weniger als 100 nm und mindestens einen aus fettlöslichen Polymeren mit Kohlenwasserstoffstruktur und aus Polymeren mit Siloxan-Struktur ausgewählten polymeren Träger mindestens einer ultraviolette Strahlungs-Anteile absorbierenden Gruppierung enthält.

**2.** Kosmetische Zusammensetzung gemäß Anspruch 1,
dadurch **gekennzeichnet**, daß
die Nanopigmente der Metalloxide einen Durchmesser von 5 bis 50 nm aufweisen.

**3.** Kosmetische Zusammensetzung gemäß Anspruch 1 oder 2,
dadurch **gekennzeichnet**, daß
das Metalloxid Titanoxid ist.

**4.** Kosmetische Zusammensetzung gemäß jedem der Ansprüche 1 bis 3,
dadurch **gekennzeichnet**, daß
das Nanopigment der Metalloxide ein umhülltes Pigment ist, das einem oder mehreren Oberflächenbehandlungsverfahren chemischer, elektronischer, mechanochemischer oder mechanischer Art mit Verbindungen unterzogen worden ist, die aus Aminosäuren, Bienenwachs, Fettsäuren, Fettalkoholen, anionischen oberflächenaktiven Mitteln, Lecithinen, Natrium-, Kalium-, Zink-, Eisen- oder Aluminiumsalzen von Fettsäuren, Metallalkoxiden, Polyethylen, Siliconen, Proteinen, Alkanolaminen, Siliziumoxiden, Metalloxiden und aus Natriumhexametaphosphat ausgewählt sind.

**5.** Kosmetische Zusammensetzung gemäß Anspruch 4,
dadurch **gekennzeichnet**, daß
das umhüllte Nanopigment aus Metalloxiden ein Pigment aus Titanoxid ist, das mit Kieselsäure, Kieselsäure und Aluminiumoxid, Kieselsäure und Eisenoxid, Aluminiumoxid und Silicon, Aluminiumoxid, Aluminiumoxid und Aluminiumstearat, Aluminiumoxid und Aluminiumlaurat, Eisenoxid und Eisenstearat, Zinkoxid und Zinkstearat, Kieselsäure und Aluminiumoxid und Silicon, Kieselsäure und Aluminiumoxid und Aluminiumstearat und Silicon, Triethanolamin, Stearinsäure oder mit Natriumhexametaphosphat umhüllt ist.

**6.** Kosmetische Zusammensetzung gemäß jedem der Ansprüche 1 bis 5,
dadurch **gekennzeichnet**, daß
sie 0,1 bis 15, vorzugsweise 0,5 bis 10, Gew.%, bezogen auf das Gesamtgewicht der Zusammensetzung, mindestens eines Nanopigments aus Metalloxiden enthält.

**7.** Kosmetische Zusammensetzung gemäß jedem der Ansprüche 1 bis 6,
dadurch **gekennzeichnet**, daß
die fettlöslichen Polymeren mit Kohlenwasserstoffstruktur, gegebenenfalls substituierte Polymere polyethylenischer Struktur, Polymere aus Polyethylenimin, aus Chitin oder Chitosan sind, an die ultraviolette Strahlung absorbierende Moleküle über eine Zwischenstufenverbindung mit einer Ester-, Amid-, Ether-, Thioether-, Sulfonyl- oder Acylfunktion gepfropft sind.

**8.** Kosmetische Zusammensetzung gemäß jedem der Ansprüche 1 bis 6,
dadurch **gekennzeichnet**, daß
die fettlöslichen Polymere mit Kohlenwasserstoffstruktur Polymere sind, die sich aus der Homo- oder Copolymerisation von ultraviolette Strahlung absorbierenden Molekülen ergeben, welche eine ungesättigte Gruppierung aufweisen, die aus den Resten ausgewählt ist: Allyl, Vinyl,, Acrylamid, Methylacrylamid, Vinyloxycarbonylmethyl, Acrylamidoalkyl und insbesondere Acrylamidomethyl, Methacrylamidoalkyl, Acrylamido(phenyl)alkyl, Methacrylamido(phenyl)alkyl, Acryloxy, Acryloxyaklyl und Acryloxypolyoxethylen.

**9.** Kosmetische Zusammensetzung gemäß Anspruch 8,
dadurch **gekennzeichnet**, daß
sich die fettlöslichen Polymeren mit Kohlenwasserstoffstrutkur aus der Homo- oder Copolymerisation von UV-Strahlung absorbierenden Molekülen, die eine ungesättigte Gruppe aufweisen, mit weiteren ungesättigten Monomeren ergeben, ausgewählt aus Acryl-, Methacryl-, Itacon-Crotonsäuren oder deren Estern, Acrylamid und seinen Derivaten, Methacrylamid und seinen Derivaten, Acrylnitril, Methacrylnitril, Styrol, $\alpha$-Methylstyrol, Isopren, Butadien, Ethylen, Propylen, Vinylestern, Vinylchloriden und - fluoriden,

Vinylidenchlorid, N-Vinylpyrrolidon, N-Methacryloyl-D-glucosamin und aus Monoestern und Diestern von Malein- und Fumrasäuren.

**10.** Kosmetische Zusammensetzung gemäß jedem der Ansprüche 7 bis 9,
dadurch **gekennzeichnet**, daß
die ultraviolette Strahlung absorbierenden Moleküle ausgewählt sind aus: Benzylidenkampfer und seinen am benzolischen Kern substituierten Derivaten, gegebenenfalls am benzolischen Kern substituierten Isophthalylidenkampfer und Terephthalylidenkampfer, gegebenenfalls mit einer oder mehreren Niedrigalkoxygruppen substituierter Zimtsäure und ihren Estern, Salycylsäure und ihren Estern, Benzoesäure und ihren Estern, p-Aminobenzoesäure und ihren an der Aminogruppe alkylierten Derivaten sowie deren Estern, gegebenenfalls substituierten Hydroxybenzophenonen, gegebenenfalls substituiertem Dibenzoylmethan, Benztriazol und 2-Arylbenztriazolen, 2-Arylbenzimidazolen, 2-Arylbenzofuranen, 2-Arylbenzoxazolen, 2-Arylindolen, Mono- oder Diphenylcyanacrylaten und aus Absorbern mit Coumarin-Struktur.

**11.** Kosmetische Zusammensetzung gemäß Anspruch 8,
dadurch **gekennzeichnet**, daß
sie einen Poly(4'-acrylamidomethyl-3-benzylidenkampfer) enthält.

**12.** Kosmetische Zusammensetzung gemäß jedem der Ansprüche 1 bis 11,
dadurch **gekennzeichnet**, daß
sie ein Polymer mit Siloxan-Struktur aus einem Diorganopolysiloxan enthält, das in seinem Molekül mindestens eine Einheit der Formel aufweist:

$$X-\overset{R'_a}{\overset{|}{Si}}-O_{\frac{3-a}{2}} \qquad (VI)$$

worin gilt:
R' bedeutet eine gesättigte oder ungesättigte $C_{1-30}$-Kohlenwasserstoffgruppe, eine halogenierte $C_{1-8}$-Kohlenwasserstoffgruppe oder eine Trimethylsilyloxygruppe,
a = 1 oder 2,
X = -A-Y,
worin A ein aliphatischer oder aromatischer zweiwertiger Kohlenwasserstoffrest mit mindestens zwei Kohlenstoffatomen ist, der gegebenenfalls ein oder mehrere Sauerstoffatome aufweist, und
Y den Rest eines Moleküls darstellt, das die UV-Strahlung filtert.

**13.** Kosmetische Zusammensetzung gemäß Anspruch 12,
dadurch **gekennzeichnet**, daß
das Diorganopolysiloxan zusätzlich Einheiten mit der Formel umfaßt:

$$R'_b-Si\,O_{\frac{4-b}{2}} \qquad (VII) \qquad \text{und} \qquad Z-\overset{R'_a}{\overset{|}{Si}}-O_{\frac{3-a}{2}} \qquad (VIII)$$

worin R' und a die in Anspruch 12 angegebenen Bedeutungen haben, b eine ganze Zahl gleich 1, 2 oder 3 ist, Z = -O-Y, worin Y dieselbe Bedeutung wie in Anspruch 12 hat, und wobei mindestens 40% der Anzahl der Reste R' den Methylrest bedeuten.

**14.** Kosmetische Zusammensetzung gemäß Anspruch 12 oder 13,
dadurch **gekennzeichnet**, daß
der Rest Y eines die ultraviolette Strahlung filternden Moleküls ein Rest von Benzylidenkampfer, der am

benzolischen Kern mit einem Hydroxyl-, $C_{1-6}$-Alkyl- oder -Alkoxyrest substituiert ist oder nicht, ein Rest von $C_{1-8}$-Dialkylbenzalmalonat,der am benzolischen Kern mit Hydroxy-, $C_{1-6}$-Alkyl- oder -Alkoxyresten substituiert ist oder nicht, ein 2-(2'-Hydroxyphenyl)benztriazolrest, der nicht substituiert ist oder an einem der aromatischen Kerne $C_{1-8}$-Alkyl-,$C_{2-8}$-Alkenyl-, Halogen-, Alkoxy-, Carboxy-, Hydroxy- oder Amino-Substituenten aufweist, ein Dibenzoylmethan-Rest, der nicht substituiert ist oder $C_{1-8}$-Alkyl- oder -Alkoxy- oder Hydroxy-Substituenten aufweist, ein Benzophenon-Rest, der nicht substituiert ist oder $C_{1-8}$-Alkyl- oder -Alkoxy- oder Hydroxy-Substituenten aufweist, ein Benzoat-Rest ist, der mit Hydroxy-, $C_{1-6}$-Alkoxy-, Amino- oder Mono- oder Di($C_{1-6}$-alkyl)aminoresten substituiert ist.

**15.** Kosmetische Zusammensetzung gemäß jedem der Ansprüche 12 bis 14,
dadurch **gekennzeichnet**, daß
sie ein Polydimethylsiloxan mit gepfropfte(m)(n) 2-(3'-Trimethylen-5'-methyl-2'-hydroxyphenyl)-benztriazol-Rest(en) enthält.

**16.** Kosmetische Zusammensetzung gemäß jedem der Ansprüche 12 bis 14,
dadurch **gekennzeichnet**, daß
sie eine Mischung aus Polydimethylsiloxanen mit gepfropften 4'-Trimethylenoxy-3-benzylidenkampfer- und 4'-Oxy-3-benzylidenkampfer-Resten enthält.

**17.** Kosmetische Zusammensetzung gemäß jedem der Ansprüche 1 bis 16,
dadurch **gekennzeichnet**, daß
sie 0,1 bis 15, vorzugsweise 0,5 bis 10, Gew.%, bezogen auf das Gesamtgewicht der Zusammensetzung, mindestens eines fettlöslichen polymeren Filterstoffes mit Kohlenwasserstoff- oder Siloxan-Struktur enthält.

**18.** Kosmetische Zusammensetzung gemäß jedem der Ansprüche 1 bis 17,
dadurch **gekennzeichnet**, daß
das Gewichtsverhältnis Nanopigment(e)/polymere(r) Filterstoff(e) 0,1 bis 10 und vorzugsweise 0,5 bis 5 beträgt.

**19.** Kosmetische Zusammensetzung gemäß jedem der Ansprüche 1 bis 18,
dadurch **gekennzeichnet**, daß
sie eine Zusammensetzung zum Schutz der menschlichen Haut oder ein Sonnenschutzmittel darstellt und in Form einer Lotion, verdickten Lotion, eines Gels, Öls, einer bläschenartigen Dispersion, einer Creme, Milch, eines Puders, Feststoffstäbchens, Schaums oder eines Spray-Produkts vorliegt.

**20.** Kosmetische Zusammensetzung gemäß jedem der Ansprüche 1 bis 18,
dadurch **gekennzeichnet**, daß
sie eine Zusammensetzung zum Schminken der Wimpern, Augenbrauen oder der Haut darstellt und in fester oder pasteuser, wasserfreier oder wässriger Form einer Emulsion, Suspension oder bläschenartigen Dispersion vorliegt.

**21.** Kosmetische Zusammensetzung gemäß jedem der Ansprüche 1 bis 18 zur Verwendung zum Schutz der Haare vor ultravioletten Strahlen,
dadurch **gekennzeichnet**, daß
sie in Form eines Shampoo, einer Lotion, eines Gels oder einer Zusammensetzung zur Spülung, zur Aufbringung vor oder nach einer Shampoonierung, vor oder nach einer Färbung oder Entfärbung, vor, bei oder nach einer Dauerwelle oder einem Ausfrisieren, in Form einer Lotion oder eines Gels zum Frisieren oder Behandeln, einer Lotion oder eines Gels zum Bürsten oder zur Wellengebung, eines Lacks für die Haare, einer Zusammensetzung zur Dauerwelle oder zum Ausfrisieren, zur Färbung oder Entfärbung der Haare vorliegt.

**22.** Kosmetische Zusammensetzung gemäß jedem der Ansprüche 19 bis 21,
dadurch **gekennzeichnet**, daß
sie zusätzlich kosmetische Hilfsstoffe enthält, ausgewählt aus Fettkörpern, organischen Lösungsmitteln, Siliconen, Verdickungsmitteln, weichmachenden Mitteln, solaren Filterstoffen für UV-A, UV-B oder eine lange Wellenbande, Antischaummitteln, hydratisierenden Mitteln, Parfüm-Produkten, Konservierungsstofen, oberflächenaktiven Mitteln, Beladungsmitteln, Sequestriermitteln, anionischen, kationischen, nicht-ionischen oder amphoteren Polymeren oder aus deren Mischungen, Treibmitteln, alkalisch oder sauer machenden Mitteln, Färbemitteln und aus Pigmenten von Metalloxiden mit einer Korngröße von 100 bis 20000 nm.

**23.** Kosmetisches Verfahren zum Schutz der menschlichen Haut und der Haare vor ultravioletter Strahlung von Wellenlängen von 280 bis 400 nm,
dadurch **gekennzeichnet**, daß
man auf die Haut oder die Haare eine wirksame Menge einer kosmetischen Zusammensetzung gemäß jedem der Ansprüche 1 bis 22 aufträgt.